# EUROPEAN PATENT APPLICATION

(11) **EP 3 646 775 A1**
(43) Date of publication of application: **06.05.2020**
(21) Application number: 18203097.3
(22) Date of filing: 29.10.2018
(51) Int. Cl.: A61B 5/00, A61B 8/00, G01N 29/24, G01N 21/17

(54) **PROBE AND SYSTEM FOR OPTOACOUSTIC IMAGING AND METHOD FOR CONTROLLING SUCH A PROBE**

(71) Applicant: iThera Medical GmbH, 81379 München (DE)
(72) Inventor: MORSCHER, Stefan, 81543 München (DE); KONRADL, Josef, 80807 München (DE); URICH, Alexander, 81477 München (DE); LONGO, Antonia, 80538 München (DE); SMOLE, Patricia, 81479 München (DE)
(74) Representative: Linsmeier, Josef

(57) **Abstract**

The present invention relates to a probe and a system for optoacoustic imaging of an object as well as a method for controlling such a probe. An irradiation unit of the probe is configured to emit electromagnetic radiation and to irradiate the object with the electromagnetic radiation, and a detection unit of the probe is configured to detect primary acoustic waves emitted by the object in response to irradiating the object with the electromagnetic radiation. The probe further comprises a couplant compartment enclosing an acoustic coupling medium between the detection unit and the irradiation unit, on the one hand, and the object, on the other hand, the couplant compartment comprising a shielding element arranged between the object and the detection unit and being configured to at least partially transmit acoustic waves and to at least partially reflect and/or absorb electromagnetic radiation, the shielding element separating the couplant compartment into a first sub-compartment and a second sub-compartment such that electromagnetic radiation emitted by the irradiation unit can pass the first sub-compartment to impinge on the object but is at least partially hindered by the shielding element to enter the second sub-compartment, and primary acoustic waves emitted by the object can pass the shielding element and the second sub-compartment to impinge on the detection unit.

## Description

The present invention relates to a probe and a system for optoacoustic imaging of an object and to a method for controlling such a probe.

Optoacoustic signal generation is based on the optoacoustic effect, according to which ultrasonic waves are generated due to absorption of electromagnetic radiation by an object, e.g. a biological tissue, and a subsequent thermoelastic expansion of the object. Excitation radiation, for example non-ionizing laser light or radiofrequency radiation, can be pulsed radiation or continuous radiation with varying amplitude or frequency. This provides optical contrast at resolutions not limited by diffraction in the optical regime, giving rise to substantially improved resolution at depth. Because the absorption of electromagnetic radiation in tissue is usually a function of physiological properties, for example hemoglobin concentration or oxygen saturation, the optoacoustic effect is predestinated for utilization in medical imaging.

It is an object of the invention to improve optoacoustic imaging of an object, in particular to provide a probe for optoacoustic imaging which allows for high signal-to-noise ratios. In particular, it is an object of the invention to reduce artifacts in obtained optoacoustic images.

This object is solved by the probe and the system for optoacoustic imaging as well as the method for controlling such a probe according to the independent claims.

The probe for optoacoustic imaging of an object according to an aspect of the invention comprises an irradiation unit configured to emit electromagnetic radiation and to irradiate the object with the electromagnetic radiation, and a detection unit configured to detect primary acoustic waves emitted by the object in response to irradiating the object with the electromagnetic radiation. The probe further comprises a couplant compartment enclosing an acoustic coupling medium between the detection unit and the irradiation unit on the one hand and the object on the other hand, the couplant compartment comprising a shielding element arranged between the object and the detection unit and being configured to at least partially transmit acoustic waves and to at least partially reflect and/or absorb electromagnetic radiation, the shielding element separating the couplant compartment into a first sub-compartment and a second sub-compartment such that electromagnetic radiation emitted by the irradiation unit can pass the first sub-compartment to impinge on the object but is at least partially hindered by the shielding element to enter the second sub-compartment, and primary acoustic waves emitted by the object can pass the shielding element and the second sub-compartment to impinge on the detection unit.

The system for optoacoustic imaging according to another aspect of the invention comprises a probe according to an aspect of the invention and a processing unit configured to derive image data regarding at least one property the object based on the detected primary acoustic waves.

According to yet another aspect of the invention a method for controlling a probe for optoacoustic imaging of an object is provided, wherein the probe comprises a couplant compartment enclosing an acoustic coupling medium between a detection unit and the irradiation unit on the one hand and the object on the other hand, the couplant compartment comprising a shielding element arranged between the object and the detection unit and being configured to at least partially reflect and/or absorb electromagnetic radiation, the shielding element separating the couplant compartment into a first sub-compartment and a second sub-compartment, and wherein the method comprises the following steps: controlling the irradiation unit to emit electromagnetic radiation and to irradiate the object with the electromagnetic radiation such that the electromagnetic radiation passes the first sub-compartment to impinge on the object but is at least partially hindered by the shielding element to enter the second sub-compartment; and controlling the detection unit to detect primary acoustic waves emitted by the object in response to irradiating the object with the electromagnetic radiation which pass the shielding element and the second sub-compartment to impinge on the detection unit.

A preferred aspect of the invention is based on the approach of reducing so-called parasitic signals, i.e. optoacoustic signals that are generated outside of an object to be imaged, by separating the space between an irradiation unit for irradiating an object with electromagnetic radiation and a detection unit for detecting acoustic waves generated in the object in response thereto on the one hand and the object on the other hand by means of a shielding element into a first sub-compartment and a second sub-compartment. In particular, the shielding element may be arranged such that it shields the detection unit from electromagnetic radiation emitted by the irradiation unit and/or reflected by the object. For example, the shielding element may be arranged in a couplant compartment holding a coupling medium between the irradiation unit and the detection unit on the one hand and the object on the other hand such that the shielding element at least partially runs in between the irradiation unit and the detection unit and thereby separates the detection unit from the irradiation unit. By this means, a direct and/or indirect irradiation, i.e. directly after being emitted by the irradiation unit or after being reflected e.g. at a surface of the object, respectively, of the detection unit with the electromagnetic radiation and a subsequent generation of unwanted (parasitic) secondary acoustic waves in the detection unit may be prevented in a simple yet reliable manner. In particular, by arranging the shielding element in the couplant compartment, the impingement of electromagnetic radiation on the detection unit and a subsequent generation of secondary acoustic waves propagating on a (active) detection surface of the detection unit can be reliably prevented.

In particular, by arranging the shielding element in the couplant compartment accordingly, artifacts in optoacoustic images due to secondary acoustic waves detected by the detection unit can be reliably prevented or at least reduced. For example, such artifacts generally appear in optoacoustic images reconstructed from detection signals generated by the detection unit upon detection of acoustic waves resulting from a superposition of the primary acoustic waves (generated in and emitted by the object) and the secondary acoustic waves (generated in the optoacoustically active detection surface of the detection unit). The present invention allows in a simple yet reliable manner to prevent the generation of or at least filter out said secondary acoustic waves.

Preferably, the irradiation unit is arranged at least partially in the first sub-compartment and the detection unit is arranged at least partially in the second sub-compartment. Accordingly, the shielding element may be configured to at least partially transmit acoustic waves generated in the object such that said waves can pass the second sub-compartment after passing the shielding element and impinge on the detection unit. The shielding element is preferably configured to further at least partially reflect and/or absorb electromagnetic radiation impinging on the shielding element directly and/or indirectly.

For example, the shielding element may comprise an optically reflecting foil or a thin optoacoustic transmitter, ideally with a very low acoustic attenuation impedance and speed of sound matched to a coupling medium in the couplant compartment. Preferably the shielding element has a high optical reflectivity at the frequency of the electromagnetic radiation, e.g. in the near infrared range (NIR), in particular of up to 97 %. For instance, the shielding element may be fabricated from metallized plastic foil, e.g. plastic foil coated with more than one and less than 5 layers of either aluminum, gold or silver, aluminum foil, or thin gold or silver foils. The shielding element preferably exhibits a thickness that allows for transmitting of acoustic waves, e.g. a thickness between 5 and 25 µm, preferably between 10 and 20 µm, in particular between 12 and 16 µm. The interplay of thickness and refractive indices of the individual layers allow adjustment of transmission and reflection properties.

In order to achieve a precise and fixed arrangement of the shielding element, the probe preferably comprises a solid coupling material, e.g. gel wax, agar, zerdine and/or the like, contained in the couplant compartment. In particular, the solid coupling material may be designed to fix the position, in particular the orientation relative to the detection unit and/or the irradiation unit, of the shielding element in the couplant compartment.

Likewise, it is possible for the shielding element to be fixed inside the couplant compartment by means of either clamps or a mounting frame, which enables the use of liquid coupling agents such as water, heavy water (D₂O) or oil. The mounting could then be adjustable by means of a millimeter screw or a small, motorized positioning stage to account for different imaging scenarios and enable modifications by the user. In particular, the optimal arrangement of the shielding element may be determined by the surface properties of the object such as refractance index, optical scattering coefficient and optical absorption coefficient.

Electromagnetic radiation emitted by the irradiation unit for example at least partially traverses the first sub-compartment to impinge on the object, where primary acoustic waves are generated in response thereto. Said primary acoustic waves then may at least partially traverse the first sub-compartment, pass the shielding element and partially traverse the second sub-compartment to impinge on the detection unit, which in response generates according detection signals based on which an optoacoustic image of the object may be obtained. Electromagnetic radiation emitted by the irradiation unit which impinges on the shielding element is at least partially reflected, e.g. towards the object and/or lateral walls of the couplant compartment.

In summary, the present invention allows for improved optoacoustic imaging of an object, in particular to provide a probe for optoacoustic imaging which allows for high signal-to-noise ratios. In particular, by means of the invention a reduction of artifacts in obtained optoacoustic images may be achieved.

In an embodiment, the shielding element is arranged and/or configured to at least partially deflect and/or direct secondary acoustic waves, which are emitted by the shielding element and/or any other element located outside of the object in response to electromagnetic radiation impinging thereon and/or which are scattered and/or reflected by any other element located outside of the object, away from the detection unit to reduce or avoid secondary acoustic waves to impinge on the detection unit. By this means, the quality of obtained optoacoustic images based on detection signals generated by the detection unit may be further enhanced, in particular by reliably prevent the secondary acoustic waves from impinging on the detection unit such that substantially no superposition of primary and secondary acoustic waves at the detection unit occurs.

For example, the secondary acoustic waves may be generated in components of the probe, e.g. lateral walls of the couplant compartment for containing the coupling medium, a coupling element for contacting a surface of the object and/or the shielding element itself upon irradiation with the electromagnetic radiation, in particular after the electromagnetic radiation is reflected multiple times inside the couplant compartment, in particular the first sub-compartment. The shielding element may be particularly shaped to direct the secondary acoustic waves, which would otherwise generate a parasitic signal in the detection unit overlapping, in particular distorting, detection signals caused by primary acoustic waves, at least partially away from the detection unit.

To this end, the shielding element may comprise an optoacoustic transmitter, e.g. a material configured to at least partially absorb the electromagnetic radiation and emit corresponding secondary electromagnetic waves in a controlled manner, in particular with a propagation direction directed away from the detection unit.

In another embodiment, the shielding element has a planar shape and/or is slanted with respect to a surface of the object and/or a perpendicular axis on the surface of the object and/or a lateral wall of the couplant compartment. For example, the shielding element comprises a plate or sheet, preferably spanning the space between the irradiation unit and the detection unit on the one hand and the object on the other hand. In particular, the shielding element may be formed by a thin foil, in particular metal foil, spanned in the couplant compartment. By this means, the shielding element can be easily and precisely arranged in order to shield the detection unit from the electromagnetic radiation emitted by the irradiation unit and/or deflect secondary waves away from the detection unit. Further, by providing a planar shaped and slanted shielding element, the propagation direction of secondary acoustic waves emitted by shielding element can be advantageously be affected, in particular reliably directed away from the detection unit. Alternatively, or in addition to the shielding element being slanted, it can follow a spherical shape that allows the generated photoacoustic wave to be directed towards a focal point that preferably lies outside the object and does not coincide with the detectors sensitivity field.

Preferably, the shielding element is slanted with respect to a surface of the object and/or a perpendicular axis on the surface of the object and/or a side wall of the coupling compartment such that the cross-section of the first sub-compartment, in particular parallel to the surface of the object and/or to the proximal or distal end of the couplant compartment, is a function of the distance to the irradiation unit and/or the detection unit, wherein the cross-section in the vicinity of the distal end of the couplant compartment is larger than the cross-section in the vicinity of the proximal end of the couplant compartment. In other words, by providing a slanted shielding device, the first sub-compartment may have a funnel-like or tapered form towards the proximal end of the couplant compartment, respectively. By this means, the electromagnetic radiation can be emitted in a divergent manner by the irradiation unit, for example in form of the radiation cone, in order to reliably and/or homogeneously induce the generation of primary acoustic waves in the region of interest the object.

In yet another embodiment, the probe further comprises one or more acoustic trap elements arranged and/or configured to at least partially absorb secondary acoustic waves, which are emitted by the shielding element and/or any other element located outside of the object in response to electromagnetic radiation impinging thereon and/or which are scattered and/or reflected by any other element located outside of the object, to reduce or avoid secondary acoustic waves to impinge on the detection unit. For example, the one or more acoustic trap elements are arranged and/or configured to attenuate, in particular damp, secondary acoustic waves impinging on the one or more acoustic trap elements, e.g. by dissipating the acoustic energy. Thus, the quality of the optoacoustic images obtained from detection signals of the detection unit can be further enhanced, for less parasitic signals due to secondary acoustic waves impinging on the detection unit will be generated.

Preferably, the one or more acoustic trap elements are fabricated at least partially from rock wool, glass fibers, textile, foam and/or thin polystyrene. In particular, the one or more acoustic trap elements may be formed by a coating of one or a combination of at least two of said materials in at least a section of an inner surface of the couplant compartment, in particular in at least a section of lateral walls of the couplant compartment and/or a section of the wall at a proximal end of the couplant compartment. By this means, reliable absorption of secondary acoustic waves emitted by the shielding element or any other and located outside of the object can be achieved, in particular largely independent of the propagation direction of said secondary acoustic waves.

In yet another embodiment, the one or more acoustic trap elements are provided at an inner surface of the couplant compartment. For example, the one or more acoustic trap elements can be arranged on lateral walls of the couplant compartment. Preferably, the one or more acoustic trap elements at least partially form the lateral walls and/or a proximal wall opposite of the couplant compartment of the object. By this means, a large surface coverage with respect to an at least partial absorption of secondary acoustic waves can be achieved, i.e. the probability of secondary acoustic waves being absorbed before impinging on the detection unit can be significantly increased. Further, the propagation of secondary acoustic waves along, in particular within, the lateral walls may be prevented or at least reduced, further limiting interaction of secondary (surface) acoustic waves with the detection unit. It is preferred to decouple and/or isolate the probe housing from potential acoustic sources or transmitters to minimize the influence of secondary acoustic waves on the detector. In particular, this relates to isolating materials mentioned as materials for acoustic traps that hinder the transmission of acoustic waves from the probe casing to the housing of the acoustic detector.

In yet another embodiment, the probe further comprises one or more optoacoustic trap elements arranged and/or configured to emit secondary acoustic waves in response to electromagnetic radiation impinging thereon such that a predominant part of the emitted secondary acoustic waves does not propagate towards and/or impinge on the detection unit. For instance, the one or more acoustic trap elements may be shaped to emit the secondary acoustic waves away from the detection unit. By this means, the generation of parasitic signals by the detection unit upon secondary acoustic waves impinging on the detection unit can be further reduced.

For example, the one or more optoacoustic trap elements may be triangular shaped or exhibit a semi-spherical shape. In particular, the one or more optoacoustic trap elements may at least partially have a curved surface, wherein the at least one or more triangular, semi-spherically or curved optoacoustic trap elements are preferably oriented with respect to the detection unit such that a surface area of the one or more optoacoustic trap elements facing the detection unit is minimized, in particular such that a major part, e.g. more than 50 %, preferably more than 65 %, in particular more than 75 %, of the surface of the one or more optoacoustic trap elements faces away from the detection unit. This may provide emitting any secondary acoustic waves away from the detection unit in a particularly simple and reliable manner.

The one or more optoacoustic trap elements may be fabricated at least partially from rock wool, glass fibers, textile, foam and/or thin polystyrene. Preferably, the one or more of the acoustic trap elements are fabricated from a soft material having a very low efficiency for conversion of electromagnetic radiation into (secondary) acoustic waves, thereby reliably reducing the total amount of secondary acoustic waves emitted.

In yet another embodiment, the one or more optoacoustic trap elements are provided at an inner surface of the couplant compartment, in particular on top of the one or more acoustic trap elements being provided at the inner surface of the couplant compartment. For example, the one or more optoacoustic trap elements may be arranged on a lateral walls and/or a proximal wall of the couplant compartment, in particular on a coating of said walls forming the one or more acoustic trap elements. By this means, secondary acoustic waves emitted by the one or more acoustic trap elements may be at least partially absorbed, in particular attenuated or damped, respectively, immediately and thereby very reliably after their generation, in particular before they might impinge on the detection unit.

Preferably, the one or more optoacoustic trap elements are shaped such that secondary acoustic waves are emitted towards the one or more acoustic trap elements. For example, the one or more optoacoustic trap elements are preferably oriented with respect to the one or more acoustic trap elements such that a surface area of the one or more optoacoustic trap elements facing one or more acoustic trap elements is maximized, in particular such that a major part, e.g. more than 50 %, preferably more than 65 %, in particular more than 75 %, of the surface of the one or more optoacoustic trap elements faces towards the one or more acoustic trap elements. This may provide absorption of secondary acoustic waves in a particularly reliable manner.

In yet another embodiment, the shielding element comprises or is designed as a semi-transparent optical element being arranged and/or configured to at least partially transmit electromagnetic radiation emitted by the irradiation unit to impinge on the object, and to reflect and/or absorb electromagnetic radiation which has been reflected and/or scattered by the object and/or any other element located outside of the object. In other words, the shielding element or at least a part thereof may form or act as a semi-transparent mirror which for instance allows electromagnetic radiation emitted by the irradiation unit to exit the couplant compartment but prevents reflected electromagnetic radiation to enter the couplant compartment, in particular the second sub-compartment. By this means, indirect irradiation of the detection unit by the electromagnetic radiation can be reliably prevented or at least reduced.

For example, the shielding element comprises a coating, e.g. disposed on a coupling element contacting the object with the probe, i.e. forming an interface between the couplant compartment and the object. In particular, the shielding element may comprise a coated membrane or foil, wherein the membrane or foil can be formed by at least a part of the coupling element. By this means, parasitic signals generated by the detection unit due to reflected electromagnetic radiation impinging on the detection unit can be reliably prevented or at least further reduced.

The coating, in particular the coating on the membrane or foil, is preferably designed to minimize absorption of reflected electromagnetic radiation in order to minimize the generation of secondary acoustic waves. In particular, the shielding element, preferably the coating on the foil or membrane, comprises a material having a high reflection and transmission coefficient and a low absorption coefficient, e.g. an absorption coefficient of 5 % or less, preferably 2 % or less, in particular 1 % or less.

In yet another preferred embodiment, the irradiation unit is configured to emit polarized electromagnetic radiation, and the shielding element comprises or is designed as a polarizing filter being arranged and/or configured to at least partially transmit the polarized electromagnetic radiation emitted by the irradiation unit to impinge on the object, and to reflect and/or absorb electromagnetic radiation which has been reflected and/or scattered by the object and/or by any other element located outside of the object. By this means, generation of parasitic signals generated by the detection unit due to reflect the electromagnetic radiation impinging on the detection unit can be easily prevented or at least further reduced.

For example, the shielding element may comprise a polarizing filter disposed on a coupling element for contacting the object such that electromagnetic radiation having the according polarization may pass the coupling element and leave the couplant compartment. A part of said electromagnetic radiation reflected outside of the couplant compartment, in particular at the surface of the object, which is thereby subjected to a polarization change and would have to pass through the polarizing filter in order to reenter the couplant compartment and impinge on the detection unit, is however at least partially absorbed at the coupling element. This can provide easy means to further prevent the generation of parasitic signals by the detection unit.

In yet another preferred embodiment, the separation of the compartments may be realized by adding scattering compounds as described before to the "dark" sub-compartment, which achieves a similar effect than the shielding element by reducing the electromagnetic radiation on the detector surface through an increase of scattering events.

In yet another embodiment, the irradiation unit and the detection unit are provided at a proximal end of the couplant compartment, and the shielding element is at least partially provided at a distal end of the couplant compartment, which is opposite to the proximal end of the couplant compartment. For example, the irradiation unit and the detection unit form at least a part of the proximal wall of the couplant compartment. Alternatively or additionally, the shielding element or at least a part thereof, for example a coated membrane or foil, may at least partially form the coupling element for contacting the object. By this means, the shielding element advantageously not only prevents, in particular reflected, electromagnetic radiation to impinge on the detection unit, but also suppresses or at least reduces the generation of secondary acoustic waves within the couplant compartment in a simple and reliable manner.

Further advantages, features and examples of the present invention will be apparent from the following description of following figures:
- Fig. 1: shows an example of a system for optoacoustic imaging of an object;
- Fig. 2: shows an example of a probe for optoacoustic imaging of an object; and
- Fig. 3: shows another example of a probe for optoacoustic imaging of an object.

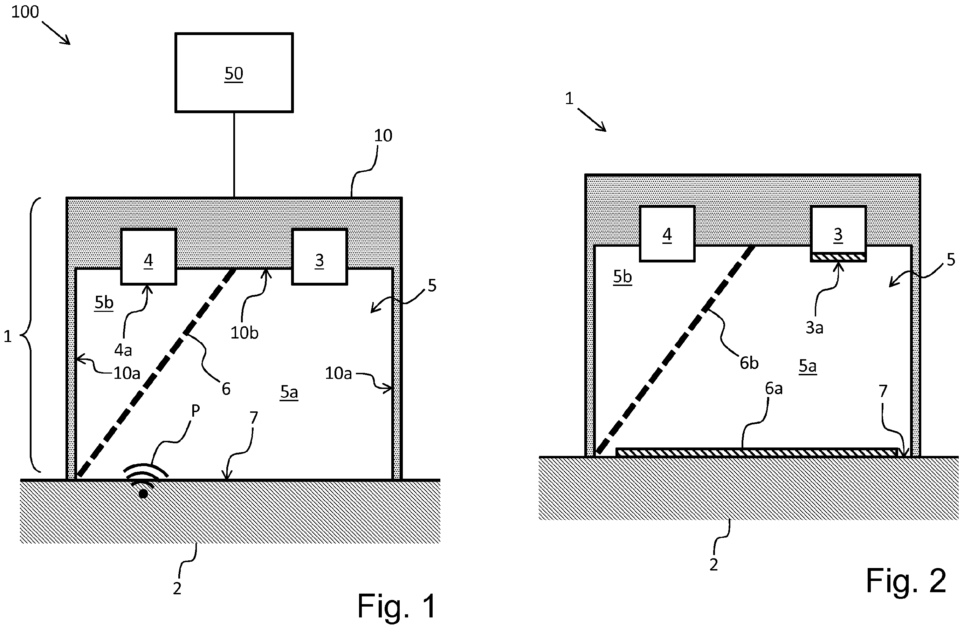

**Figure 1** shows an example of a system 100 for optoacoustic imaging of an object 2 comprising a probe 1 and a processing unit 50. The probe 1 has an irradiation unit 3 for irradiating the object 2 with electromagnetic radiation, a detection unit 4 for detecting primary acoustic waves P generated in the object 2 in response to irradiating the object with the electromagnetic radiation, a couplant compartment 5 containing a coupling medium for acoustically coupling the object 2 and the detection unit 4 and a shielding element 6 separating the couplant compartment 5 into a first sub-compartment 5a and a second sub-compartment 5b. The processing unit 50 is configured to generate image data regarding properties of the object 2 based on the primary acoustic waves P detected by the detection unit 4.

The detection unit 2, which is given for example by one or more, in particular an array of, ultrasonic transducers, comprises a detection surface 4a sensitive to primary acoustic waves P impinging on the detection unit 2. The detection unit 2 is configured to generate detection signals in response to the primary acoustic waves P impinging thereon. The detection signals can be transmitted to the processing unit 50, which is preferably configured to process the detection signals such that an optoacoustic image of the object 2 is reconstructed from the detection signals.

The probe 1 preferably comprises a housing 10, in particular for handheld operation, which may enclose the irradiation unit 3 and the detection unit 4 and forms and/or includes the couplant compartment 5. The coupling medium contained in the couplant compartment 5 is separated from the object 2 by a coupling element 7, e.g. a, preferably flexible, membrane, which is configured to contact the object 2 during image acquisition. In other words, the contact element 7 forms an interface between the couplant compartment 5 and the object 2.

Preferably, the housing 10 comprises lateral walls 10a as well as a proximal wall 10b of the couplant compartment 5, wherein the proximal wall 10b may be at least partially constituted by the irradiation unit 3 and/or the detection unit 4, in particular by the detection surface 4a. In other words, in present example the detection unit 4 and the irradiation unit 3 are disposed at a proximal end of the couplant compartment 5, and the couplant compartment 5 is sealed by the contact element 7 which is provided at a distal end of the couplant compartment 5.

The shielding element 6, for example a thin metal or metallized foil or membrane, which separates the coupling compartment 5 into the first and second sub-compartments 5a, 5b is configured to at least partially transmit acoustic waves and at least partially reflect electromagnetic radiation. The shielding element 6 hinders electromagnetic radiation emitted by the irradiation unit 3 into the first sub compartment 5a towards the object 2 and/or subsequently at least partially reflected at the object 2 to enter the second compartment 5b and to impinge on the detection unit 4, where it would generate an unwanted parasitic detection signal due to secondary acoustic waves generated at the detection surface 4a, thereby causing artifacts in the reconstructed optoacoustic image. At the same time, the primary acoustic waves P generated in the object 2 enter the couplant compartment 5 through the coupling element 7 and can pass the shielding element 6 and the second sub-compartment 5b to impinge on the detection unit 4 substantially unhindered.

**Figure 2** shows an example of a probe 1 for optoacoustic imaging of an object 2. In present example, the probe 1 comprises a shielding element 6a, 6b, wherein a first part 6a of the shielding element is provided at a distal end of couplant compartment 5, and a second part 6b of the shielding element separates the couplant compartment 5 into a first sub-compartment 5a and a second sub-compartment 5b (similarly to shielding element 6 shown in Figure 1).

Further, an irradiation unit 3 for emitting electromagnetic radiation towards the object 2 is arranged at least partially in the first sub-compartment 5a at a proximal end of the couplant compartment 5, and a detection unit 4 for detecting primary acoustic waves generated in the object 2 in response to the irradiation with electromagnetic radiation is at least partially located in the second sub-compartment 5b at the proximal end of the couplant compartment 5.

The second part 6b of the shielding element is configured to at least partially transmit acoustic waves and to at least partially reflect and/or absorb electromagnetic radiation (like shielding element 6 shown in Figure 1).

In distinction to this, the first part 6a of the shielding element is configured to at least partially transmit electromagnetic radiation emitted by the irradiation unit 3 to impinge on the object 2, and at the same time to reflect and/or absorb electromagnetic radiation which has been reflected and/or scattered by the object 2 and/or any other element located outside the object 2.

To this end, the first part 6a may comprise or be designed as a semi-transparent optical element at least partially separating the couplant compartment 5 from the object 2. For example, the first part 6a may comprise a coating, in particular a metal coating, disposed on coupling element 7 (cf. Figure 1). Thus, electromagnetic radiation emitted by the irradiation unit 3 traverses the first sub-compartment 5a and passes through the first part 6a of the shielding element to impinge on the object 2. However, a part of this electromagnetic radiation reflected by the object 2, in particular at the surface of the object 2, cannot reenter the couplant compartment 5 due to the reflective and/or absorptive properties of the first part 6a. Thereby, generation of secondary acoustic waves, in particular within the couplant compartment 5, which would cause the detection 4 unit to generate a parasitic (detection) signal, can be suppressed or at least reduced.

Alternatively or additionally, for example, the first part 6a may be configured to act as a polarizing filter, letting electromagnetic radiation having a first polarization pass through and blocking off electromagnetic radiation having a second polarization perpendicular to the first polarization. The irradiation unit 3 may be configured to emit polarized electromagnetic radiation, in particular having the first polarization, e.g. by means of a polarizing element 3a. Thus, the electromagnetic radiation emitted by the irradiation unit 3 may pass the first part 6a, but upon being at least partially reflected and/or scattered by the object 2, which at least partially changes polarization of the electromagnetic radiation, the electromagnetic radiation is reliably blocked off by the first part 6a.

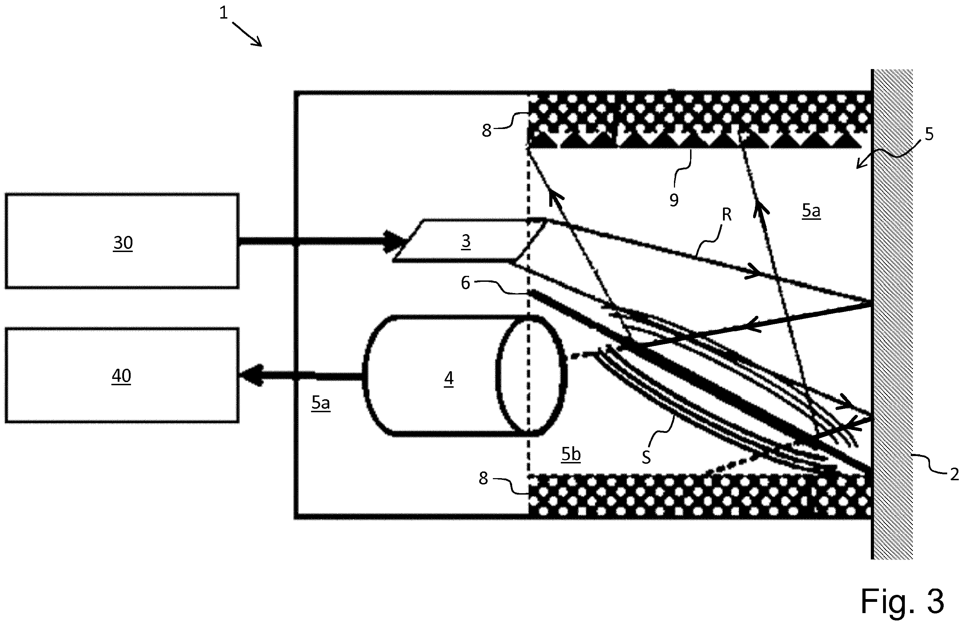

**Figure 3** shows another example of a probe 1 for optoacoustic imaging of an object 2. Similarly to the example shown in Figure 1, the probe 1 of present example comprises a detection unit 4 configured to detect primary acoustic waves generated the object 2 in response to the electromagnetic radiation R emitted by an irradiation unit 3. The probe 1 also comprises a shielding element 6 configured to at least partially reflect and/or absorb electromagnetic radiation R and to at least partially transmit acoustic waves, in particular primary acoustic waves generated in the object 2. The shielding element 6 separates a couplant compartment 5 into a first sub-compartment 5a and a second sub-compartment 5b.

Preferably, the probe 1 further comprises one or more acoustic trap elements 8 for at least partially absorbing secondary acoustic waves, e.g. secondary acoustic waves S generated in and emitted by the shielding element 6.

Alternatively or additionally, the probe 1 comprises optoacoustic trap elements 9 for at least partially absorbing electromagnetic radiation R emitted by the irradiation unit 3 and reflected in the couplant compartment 5, in particular at an interface between the probe 1 and the object 2 and/or at the object 2 and/or at the shielding element 6.

As shown in the present example, the electromagnetic radiation R, e.g. in form of an irradiation cone, emitted by the irradiation unit 3 traverses the first sub-compartment 5a and impinges on the object 2, in particular a surface of the object 2 facing the probe 1. A first, major part of the radiation R penetrates the object 2 and generates primary acoustic waves beneath the surface of the object 2, wherein the primary acoustic waves contain information, in particular image information, relating to the object 2. A second, minor part of the radiation is reflected at the interface between the probe 1 and the object 2, in particular at the surface of the object 2 and/or within the object 2. Said second part of the radiation R at least partially impinges on the shielding element 6, which is arranged at least partially in between the irradiation unit 3 and the detection unit 4, in particular slanted with respect to the surface of the object 2 and/or a perpendicular axis on the surface of the object 2 and/or the lateral walls (see 10a, 10b in Figure 1) of the couplant compartment 5.

The shielding element 6, being at least partially absorptive to the electromagnetic radiation R, absorbs at least some of the impinging second part of the electromagnetic radiation R and may thereby generate secondary acoustic waves S.

In order to avoid at least a major part of the secondary acoustic waves S emitted by the shielding element 6 to impinge on the detection unit 4, the shielding element 6 is preferably arranged and/or configured to at least partially emit and/or direct said generated secondary acoustic waves S away from the detection unit 4, in particular towards the acoustic trap elements 8, in particular towards the acoustic trap elements 8 being preferably arranged at the (lower) lateral wall of second sub-compartment 5b and/or towards the acoustic and/or optoacoustic trap elements 8, 9 being preferably arranged at the (upper) lateral wall of first sub-compartment 5a.

To this end, the shielding element 6 may preferably have a planar shape, such that in combination with the slanted orientation of the shielding element 6, no or only a minor part of the shielding element 6 faces the detection unit 4. In particular, the planar shape in combination with the slanted orientation of the shielding element 6 allows that a section of the shielding element 6, in which the second part of the electromagnetic radiation R impinges on the shielding element 6, is not facing the detection unit 4 but preferably the acoustic and/or optoacoustic trap elements 8, 9.

The acoustic trap elements 8 are arranged on lateral walls of the couplant compartment 5. Preferably, the acoustic trap elements 8 are formed by a coating disposed on the lateral walls of the couplant compartment 5. For example, the lateral walls of the couplant compartment 5 may be at least partially coated by rock wool, glass fibers, textile, foam or a thin polystyrene film or a combination thereof. While strongly attenuating, in particular damping, impinging secondary acoustic waves S, these materials also have a very low conversion efficiency, i.e. they absorb most of impinging electromagnetic radiation R without converting it into acoustic waves.

Thus, the acoustic trap elements 8 are not only configured to at least partially absorb the secondary acoustic waves S emitted by the shielding element 6, but also secondary acoustic waves S emitted by any other element located outside object 2 in response to the electromagnetic radiation R impinging thereon and/or which are scattered and/or reflected by any other element located outside of the object 2. In particular, the acoustic trap elements disposed on the lateral walls of the couplant compartment 5 at least partially suppress the reflection of secondary acoustic waves S within the couplant compartment 5. In this arrangement, the acoustic trap elements 8 also hinder secondary acoustic surface waves propagating along the lateral walls of couplant compartment 5 from reaching the detection unit 4.

The shielding element 6, being at least partially reflective to the electromagnetic radiation R, reflects at least some of the impinging second part of the electromagnetic radiation R, in particular directs some of the impinging second part of the electromagnetic radiation R towards the optoacoustic trap elements 9. If not for the shielding element 6, the second part of the electromagnetic radiation R would, at least partially, impinge on the detection unit 4 and generate secondary acoustic waves S, in particular secondary surface acoustic waves, at a detection surface of the detection unit 4, which would in turn cause the detection unit 4 to generate parasitic (detection) signals. This is indicated by the dotted lines.

The optoacoustic trap elements 9, similarly to the acoustic trap elements 8, are arranged on the lateral walls of couplant compartment 5, in particular on top of the acoustic trap elements 8. This allows an immediate absorption of secondary acoustic waves S emitted by the optoacoustic trap elements 9 by the acoustic trap elements 8 upon impinging electromagnetic radiation R, in particular some of the second part of the electromagnetic radiation R reflected at the object 2 and subsequently at the shielding element 6.

The optoacoustic trap elements 9 may comprise the same or at least similar materials as utilized in the acoustic trap elements 8. Further, the optoacoustic trap elements 9 are preferably shaped such that the emitted secondary acoustic waves S are direct away from the detection unit 4, in particular towards the acoustic trap elements 8. For example, as indicated in Figure 3, the optoacoustic trap elements 9 may have a triangular or semi-spherical shape, wherein the major part of the surface of the optoacoustic trap elements 9 faces the acoustic trap elements 8.

The probe 1, in particular the irradiation unit 3, is coupled to a radiation source 50, e.g. a laser, configured to generate the electromagnetic radiation, preferably in the near infrared range (NIR) of the electromagnetic spectrum, which is for example coupled to the irradiation unit 3 by means of a light guide, e.g. an optical fiber. The irradiation unit 3 can be formed by a distal end of said light guide. Additionally or alternatively, the irradiation unit 3 may comprise irradiation optics, e.g. for collimating, diffusing, focusing and/or directing the emitted electromagnetic radiation, in particular onto or towards the object 2, respectively. The irradiation optics are preferably configured to shape and/or direct the electromagnetic radiation in such a way that it does not, at least not directly, impinge on the detection unit 4. Alternatively or additionally, the irradiation optics are configured to shape and/or direct the electromagnetic radiation in such a way that the electromagnetic radiation does not impinge on the shielding element 6.

The probe 1 is further coupled to detection electronics 40 configured to process, at least pre-process, detection signals generated by the detection unit 4 upon primary acoustic waves impinging thereon. For example, the detection signals may be amplified, filter and/or converted to digital signals by means of the detection electronics 40. Alternatively or additionally, the detection electronics 40 may be configured to control the detection unit 4 in particular as a function of the radiation source 30, e.g. to only detect primary acoustic waves only during the predefined duration during which the irradiation unit 3 emits electromagnetic radiation.

In another embodiment (not shown), the radiation source 30 and/or the detection electronics 40 may be part of the probe 1.

## Claims

1. A probe (1) for optoacoustic imaging of an object (2), the probe (1) comprising
- an irradiation unit (3) configured to emit electromagnetic radiation (R) and to irradiate the object (2) with the electromagnetic radiation (R),
- a detection unit (4) configured to detect primary acoustic waves (P) emitted by the object (2) in response to irradiating the object (2) with the electromagnetic radiation (R), and
- a couplant compartment (5) enclosing an acoustic coupling medium between the detection unit (3) and the irradiation unit (3), on the one hand, and the object (2), on the other hand, the couplant compartment (5) comprising a shielding element (6) arranged between the object (2) and the detection unit (4) and being configured to at least partially transmit acoustic waves and to at least partially reflect and/or absorb electromagnetic radiation (R), the shielding element (6) separating the couplant compartment (5) into a first sub-compartment (5a) and a second sub-compartment (5b) such that electromagnetic radiation (R) emitted by the irradiation unit (3) can pass the first sub-compartment (5a) to impinge on the object (2) but is at least partially hindered by the shielding element (6) to enter the second sub-compartment (5b), and primary acoustic waves (P) emitted by the object (2) can pass the shielding element (6) and the second sub-compartment (5b) to impinge on the detection unit (4).

2. The probe (1) according to claim 1, the shielding element (6) being arranged and/or configured to at least partially deflect and/or direct secondary acoustic waves (S), which are emitted by the shielding element (6) and/or any other element located outside of the object (2) in response to electromagnetic radiation (R) impinging thereon and/or which are scattered and/or reflected by any other element located outside of the object (2), away from the detection unit (4) to reduce or avoid secondary acoustic waves (S) to impinge on the detection unit (4).

3. The probe (1) according to claim 1 or 2, the shielding element (6) having a planar shape and/or being slanted with respect to a surface of the object (2) and/or a perpendicular axis on the surface of the object (2) and/or a lateral wall (10a) of the couplant compartment (5).

4. The probe (1) according to any preceding claim, further comprising one or more acoustic trap elements (8) arranged and/or configured to at least partially absorb secondary acoustic waves (S), which are emitted by the shielding element (6) and/or any other element located outside of the object (2) in response to electromagnetic radiation (R) impinging thereon and/or which are scattered and/or reflected by any other element located outside of the object (2), to reduce or avoid secondary acoustic waves (S) to impinge on the detection unit (4).

5. The probe (1) according to claim 4, the one or more acoustic trap elements (8) being provided at an inner surface of the couplant compartment (5).

6. The probe (1) according to any preceding claim, further comprising one or more optoacoustic trap elements (9) arranged and/or configured to emit secondary acoustic waves (S) in response to electromagnetic radiation (R) impinging thereon such that a predominant part of the emitted secondary acoustic waves (S) does not propagate towards and/or impinge on the detection unit (4).

7. The probe (1) according to claim 6, the one or more optoacoustic trap elements (9) being provided at an inner surface of the couplant compartment (5), in particular on top of the one or more acoustic trap elements (8) being provided at the inner surface of the couplant compartment (5).

8. The probe (1) according to any preceding claim, the shielding element (6) comprising or being designed as a semi-transparent optical element (6a) being arranged and/or configured to at least partially transmit electromagnetic radiation (R) emitted by the irradiation unit (3) to impinge on the object (2), and to reflect and/or absorb electromagnetic radiation (R) which has been reflected and/or scattered by the object (2) and/or any other element located outside of the object (2).

9. The probe (1) according to any preceding claim, the irradiation unit (3) being configured to emit polarized electromagnetic radiation (R), and the shielding element (6a) comprising or being designed as a polarizing filter being arranged and/or configured to at least partially transmit the polarized electromagnetic radiation (R) emitted by the irradiation unit (3) to impinge on the object (2), and to reflect and/or absorb electromagnetic radiation (R) which has been reflected and/or scattered by the object (2) and/or by any other element located outside of the object (2).

10. The probe (1) according to claim 8 or 9, the irradiation unit (3) and the detection unit (4) being provided at a proximal end of the couplant compartment (5), and the shielding element (6) being at least partially provided at a distal end of the couplant compartment (5), which is opposite to the proximal end of the couplant compartment (5).

11. A system (100) for optoacoustic imaging of an object (2) comprising
- a probe (1) according to any one of the previous claims and
- a processing unit (50) configured to derive image data regarding at least one property the object (2) based on the detected primary acoustic waves (P).

12. A method for controlling a probe (1) for optoacoustic imaging of an object (2), the probe (1) comprising a couplant compartment (5) enclosing an acoustic coupling medium between a detection unit (4) and an irradiation unit (3), on the one hand, and the object (2), on the other hand, the couplant compartment (5) comprising a shielding element (6) arranged between the object (2) and the detection unit (4) and being configured to at least partially reflect and/or absorb electromagnetic radiation (R), the shielding element (6) separating the couplant compartment (5) into a first sub-compartment (5a) and a second sub-compartment (5b), the method comprising the following steps:
- controlling the irradiation unit (3) to emit electromagnetic radiation (R) passing the first sub-compartment (5a) to impinge on the object (2) to cause the object (2) to emit primary acoustic waves (P), wherein the emitted electromagnetic radiation (R) is at least partially hindered by the shielding element (6) to enter the second sub-compartment (5b); and
- controlling the detection unit (4) to detect primary acoustic waves (P) having passed the shielding element (6) and the second sub-compartment (5b) to impinge on the detection unit (4).
